# EUROPEAN PATENT SPECIFICATION

(11) **EP 3 265 152 B1**
(45) Date of publication and mention of the grant of the patent: **29.01.2020**
(21) Application number: 16733179.2
(22) Date of filing: 07.03.2016
(51) Int. Cl.: A61M 5/31

(54) **DATA ACQUISITON MEANS, ESPECIALLY FOR A PEN-TYPE DRUG DELIVERY DEVICES**
DATENERFASSUNGSVORRICHTUNG, INSBESONDERE FÜR EINE STIFTARTIGE ARZNEIMITTELABGABEVORRICHTUNG
MOYEN D'ACQUISITION DE DONNÉES, EN PARTICULIER POUR DES DISPOSITIFS D'ADMINISTRATION DE MÉDICAMENT DE TYPE STYLO

(30) Priority: 06.03.2015 HU P1500093; 06.03.2015 US 201562129107 P
(43) Date of publication of application: 10.01.2018
(73) Proprietor: Berey, Attila, 8000 Székesfehérvár (HU); Markovich, Péter, 1115 Budapest (HU); Tímár, Béla, 1046 Budapest (HU)
(72) Inventor: Berey, Attila, 8000 Székesfehérvár (HU); Markovich, Péter, 1115 Budapest (HU); Tímár, Béla, 1046 Budapest (HU)
(74) Representative: Szabo, Zsolt
(86) International application number: PCT/HU2016/050009
(87) International publication number: WO 2016/142727

(56) References cited:
- WO-A1-2010/098931
- WO-A1-2011/073806
- WO-A1-2013/004843
- WO-A1-2013/004844
- WO-A2-2010/062675
- WO-A2-2012/127046

## Description

The present invention relates to a data acquisition means to be used with drug delivery devices, in particular, to a data acquisition and dispensing means that can be mounted onto pen-type insulin dispensing devices.

Wide-spread use of a medical device is substantially affected by its cost and complexity. This particularly applies to people living with diabetes, the significant masses of whom can generally be considered disadvantaged as to their financial potential, especially in developing countries. Consequently, a newly developed device will only become a device that could improve their health and comfort if said device is generally a low-cost device and can optionally be used for a relatively long period of time. Moreover, the great masses of people living with diabetes (except for those having type I diabetes) are of older age, belonging generally to the age-group of above fifty, and thus most of them are unable or hardly able to use and to learn how to use advanced technological means and the most modern devices, such as e.g. computers, tablets, smartphones, etc. Therefore, beyond the aforementioned considerations, a newly developed device should have the simplest configuration and design as far as practical usage is concerned, and it should also be a device that has - within the range of possibilities - the least possible number of function buttons. A device that is cheap and easy to use in everyday life can result in effective application even amongst people living under disadvantaged conditions, and a more effective improvement in their health condition can be observed for such a device.

One of everyday tasks of people living with diabetes is insulin administering by means of insulin dispensers, e.g. pen-type insulin dispensing devices, i.e. insulin pens. Insulin administering becomes an automated routine activity in a very short time for people living with diabetes, an action that is carried out by habit, and thus they often do not remember whether or not the required insulin dose has already really been administered. A further difficulty in monitoring the administration is that in a single administration event only a small fraction of the contents within the vial of the insulin pen becomes dispensed and thus the user is unable to reliably determine by observing the amount of insulin remaining within the vial whether or not the required insulin dose has been recently administered.

International Publication Pamphlet No. WO 2012/127046 A2 discloses a data acquisition means that can be snap-fitted onto an insulin dispensing device, e.g. an insulin pen. Said means can be disposed on the actuating button of the insulin pen and it detects by a suitable sensor if the user presses the dispensing button in order to administer insulin. Accordingly, said means comprises a sensor, electronics, a power source, and optionally a transmitter and/or at least one LED. According to a preferred embodiment, said means stores data related to the instant of insulin administration and in harmony with this, a LED emitting e.g. in red indicates if time passed since the last administration is not enough. Then, when suitable amount of time has passed, the red LED switches off and a LED emitting in green is activated to indicate that the insulin pen can/should be used again. In one embodiment of said means, operation of a LED emitting in green for a given period of time indicates that administration has taken place, then said LED switches off if the given amount of time has passed. Said means is connected to the push-button of the insulin pen by a detachable connection, thus it can be dismounted from the insulin pen and/or can be simply relocated to another insulin pen, if necessary - when e.g. the power source of said means provided by a button cell runs down or the insulin pen reaches the end of its lifetime - to replace the power source. The transmitter in the cap is provided as a unit that is configured to optical or radio-frequency data transfer; it is e.g. a Bluetooth transmitter, or can be configured to be also suitable for wired data transfer.

A disadvantage of said data acquisition means is that continuous operation of the LEDs emitting in red and green is power-consuming, therefore the power source in said means runs down much faster than the lifetime of the insulin pen (i.e. the consumption of its insulin content) and thus its replacement becomes necessary. Although operating the green LED of the device for only a predetermined period of time would save energy, it does not indicate reliably the time passed since the previous administration, because this function thereof is lost when lightning of said LED is switched off. The run-down of the power source causes deletion of the insulin-dispensing data and other pieces of information regarding the operation. Thus, said means is not suitable for acquiring and storing data on a time scale comparable to the lifetime of the insulin pen. A significantly greater disadvantage of said means is, however, that in lack of suitable power management, run-down of the power source might cause said means getting abruptly inoperable, which is annoying, but can also lead to a life-threatening situation. A Bluetooth transmitter optionally applied in said means - given the capacity of the power source (button cell) thereof - cannot be considered as a low-consumption (i.e. power source sparing) device, therefore its operation (signal transmission) near the end of its power source's lifetime might cause a run-down of the power source with high probability, and thus results in unexpected inoperability of said means.

International Publication Pamphlet No. WO 2010/098931 A1 discloses a system aiding the monitoring of administering insulin or another drug, comprising a data acquisition and transmitting knob that is firmly built into a pen-type dispensing device, as well as a data management unit that receives and evaluates data transmitted by said knob and provides a response depending on the information content. The data management unit is provided by e.g. a blood glucose meter disposed in the storage case of the pen, a cellphone, a personal computer, a portable computer, an external network server or a combination thereof. The response given to the user is generated by a LED or an LCD screen arranged in the pen, or by a display or a speaker of the data management unit. The data transfer between the pen end the data management unit can take place by electromagnetic waves in the infrared, optical or radio frequency range. The pen and/or the data management unit can be provided with an RFID-reader unit suitable for reading an RFID tag e.g. on the drug vial arranged within the pen. Thus, the user could be warned - preferably before the administration - if the drug or the time of administration would be inappropriate. The electronics of the knob of the pen measure, store and transmit the time of pressing down and releasing the knob, and the strength of pressing down the knob, since the rate of drug injection also depends on the strength of pressing said knob. According to experiences, this last feature is often inappropriate, because it makes the amount of the administered drug uncontrollable. Energy management or an operation mode that realizes energy management in relation to the system is neither disclosed nor mentioned.

The object of the present invention is to eliminate or at least to alleviate disadvantages of the prior art solutions. The present invention is only limited by the claims.

In particular, an object of the present invention is to provide a low-cost data acquisition means that can be mounted onto pen-type drug delivery devices to acquire information about the operation of the drug delivery device continuously and for a long period of time, as well as to provide a signal to inform the user thereabout. Here, and from now, the term 'long time' refers basically to the lifetime of the drug delivery device that is used together with said data acquisition means. Due to the wear caused by the daily usage (even several times, in some cases), this is about one year in practice.

A further object of the invention is to provide a low-cost data acquisition means that can indicate the pieces of information about the operation of the drug delivery device to the user in compliance with the power supply of the data acquisition means, and thus it is capable of predicting an expectable inoperability of said data acquisition means to the user in advance.

A further object of the invention is to provide a low-cost data acquisition means that is capable of regularly transmitting data related to the operation of the drug delivery device to an external data receiving and/or data processing unit, as well as also preserving the transmitted information for a long period of time.

The aforementioned objects are achieved by providing a data acquisition means in accordance with claim 1 that is mountable onto a pen-type drug delivery device, preferably onto an insulin pen, and provided with data acquisition and informing functions, as well as has its own power management, i.e. said means is configured, in particular, to transmit signals related to drug delivery information of the drug delivery device for transmitting pieces of information to a user, wherein said signals are in compliance with the actual charge level of the power source and also change as said charge level of the power source changes. Preferred exemplary embodiments of the data acquisition means are set out in claims 2 to 10.

The pen-type drug delivery device is preferably an insulin pen for dispensing insulin as the drug. It is obvious, that the data acquisition means according to the invention can also be used in the case of long term regular administration of other drug(s).

The task of the data acquisition means according to the present invention is, on the one hand, to inform the user on the fact that insulin administration has already been performed and, on the other hand, to acquire pieces of information about the insulin administration itself. Informing the user is preferably achieved by LED light sources arranged within said means and emitting in red and in green, by operating said LEDs in compliance with the power content of the power source used in the data acquisition means. That is, the number of flashes by the LEDs corresponds to the actual charge level of the power source. If the insulin has already been administered, the light source of the data acquisition means will inform the user by flashing red light before actuation of the insulin pen that the required dose has already been administered. Thus, a repeated insulin administration which could result in serious health problems is avoided. This function greatly enhances the safety and comfort of patients, because he/she stands under a continuous supervision by the data acquisition means, no accidents can occur, medical condition of the patient may improve and he/she can perform his/her daily routine more comfortably.

Besides the informing function, the data acquisition means has data acquisition function as well. This comprises storing the instant and the duration of insulin administration by means of exploiting a clock built into said means. These functions facilitate the physician in his assessing the condition of the patient for the treatment of the patient. Physical condition of the patient can be monitored and assessed between examinations, thus the physician can get an accurate notion of the physical condition and its reasons as well. The administered amount can be determined form the duration of insulin administration (i.e. the length thereof), there is also a possibility to calculate it, but for evaluation purposes, knowledge of the duration of administration is sufficient, as - according to our experiences and studies - patients administer the required dose in an ingrown manner, i.e. every time substantially within the same amount of time.

The technical criteria to be fulfilled by the data acquisition means according to the invention have been established on the basis of the needs of potential users, in particular, people living with diabetes. Our aims by fulfilling the criteria established are to make the life of the patients easier, improve their condition, increase their comfort and safety, as well as to provide appropriate means for simply monitoring their disease by physicians.

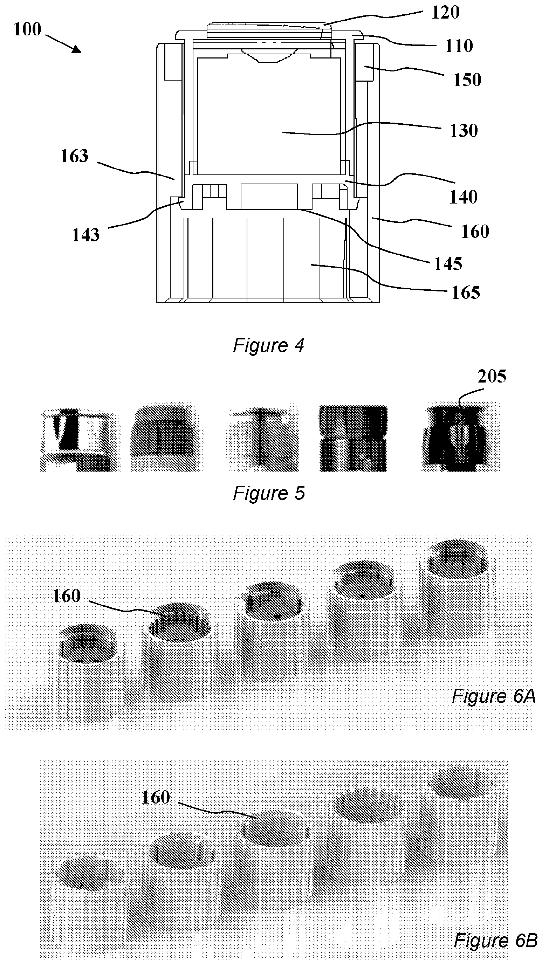

Nowadays there is a great number of commercially available pen-type drug delivery devices, in particular insulin dispensing pens, which - although serving the same function - have large differences in their appearance, external design and dimensions. The data acquisition means according to the invention has a modular construction (i.e. it is obtained by assembling several parts) and is mountable onto substantially any commercially available insulin pen (several examples are shown in Figure 5). To achieve this, only the connecting element that serves to connect the data acquisition means to the insulin pen is varied, further parts of the data acquisition means have the same construction independent of the insulin pen to be used with the data acquisition means. In particular, surface of the of said connecting element that serves for connection is constructed with different surface structures, depending on the actual commercially available insulin pen, in particular its actuator member, whereupon the connecting element (and thus the data acquisition means according to the invention) is to be mounted and fixed by a frictional and/or form-fitting connection between the connecting element and said actuator member in such a manner that said means can be removed without being damaged by applying a sufficiently large force to the data acquisition means, if necessary.

Furthermore, the data acquisition means is constructed in such a way that its actuation by the push-button and the actuation of the insulin pen itself take place by the same action, because this is the only way to guarantee that said means acquires data about the actuation and provides response to the user and the physician appropriately. In this way the amount of the administered insulin becomes calculable, it will be proportional to the duration of pressing the push-button.

Furthermore, the data acquisition means is constructed by including an own power source, as an important feature of the insulin pens is their mobility, i.e. they have to be usable anywhere and anytime with no medical help or any further requisites. The power source of its own is required for operating the electronics to provide response lights, to process and store the data acquired. A wired construction that is connected to the mains is immobile, and therefore obsolete, as it would reduce the mobility of a product. Moreover, the requirement for an external power source would also increase the size and complexity of the device, which is disadvantageous.

Furthermore, the data acquisition means is constructed in such a way, that to avoid serious consequences of a repeated administration or a lack of administration, it reminds the user for a predetermined amount of time (practically, for e.g. 3 hours) of the fact that the last insulin administration has already been performed. This enhances comfort of the users. This function is provided by a response generated by a LED light source of low power consumption that - upon intentional actuation by the user - informs/warns the user by means of a number of flashes depending on the charge level of the power source on the fact that administration has already been completed and the next administration is not actual yet.

Furthermore, the data acquisition means is constructed in such a way that the memory within the electronic subassembly stores the number, the instant and the duration of insulin administrations for a long time, preferably for the whole lifetime of the insulin pen. Physician of the patient can easily inspect and assess patient's condition from the number and duration of the pressing down of the push-button of the device, which are pieces of information acquired and stored by the electronic subassembly. Furthermore, the amount of administered insulin can be determined from a duration of the push-button being pressed down. From the number of said push- button being pressed down it can also be determined whether or not the user regularly administered the required insulin. Furthermore, optionally the amount of administered insulin can also be calculated from the duration of the push-button being pressed down and the volumetric flow rate capacity of the needle of the insulin pen. This is, however, not substantially important, the users mostly self-administer insulin for the same duration every time in an ingrown manner. In case of a deviation from the ingrown duration, the physician may precisely determine the cause of change in patient's condition.

Furthermore, the data acquisition means is constructed in such a way that when it is put into a communication storage case together with the insulin pen, the data communication between the data acquisition means and the storage case enables reading out data stored in said means related to the operation of the insulin pen. Said multifunctional storage case provides solution for reading out data acquired by the data acquisition means according to the invention and stored in the memory disposed within the data acquisition means for a long time, practically from the first drug administration after connecting said means with the actually used drug delivery device: each time when after finishing administering the drug, the pen-type drug delivery device is put back into the storage case, and the case - preferably in an automated manner, i.e. without the active contribution of the user - reads out the pieces of information stored in the data acquisition means and then preferably stores the information in its own memory unit for later usage, e.g. by a physician. The transfer is carried out through a connection of low power consumption, preferably via an infrared communication channel. The communication storage case is provided preferably by e.g. a casing that is used to store the pen when it is not in use with the appropriate electronic modifications/upgrades.

Usage of a data acquisition means according to the invention is a great help in the life and work of both the patient and the physician. Due to the automatic data communication, operation of the data acquisition means requires only little effort from both parties. The possibility to avoid by means of power supply dependent signals that said means become unexpectedly inoperable, significantly improves life quality of the patient and reliability of the use of a drug delivery device equipped with the data acquisition means according to the invention with power management function.

In what follows, the data acquisition means according to the invention, in particular, its preferred exemplary embodiments mountable onto pen-type insulin dispending devices, as well as their operation is described in detail with reference to the accompanying drawings, wherein
- Figures 1A and 1B illustrate an exemplary insulin pen and a corresponding data acquisition means according to the invention, respectively, before assembly and after assembly;
- Figures 2A and 2B show a preferred exemplary embodiment of the data acquisition means according to the invention in perspective view from the outside and from below, respectively;
- Figure 3 is an exploded view of the data acquisition means shown in Figure 2 with the parts making up the data acquisition means;
- Figure 4 is a sectional view of a possible exemplary embodiment of the data acquisition means when assembled;
- Figure 5 illustrates, without completeness, the outer construction of the actuator member used in several commercially available insulin pens (from left to right, the insulin pen marked as Luxura® from Eli Lilly®, as Solostar® from the Sanofi®, as Savivo® from Eli Lilly®, as Novopen® 4 from Novo Nordisk® and as Tactipen® from the Sanofi® company are shown);
- Figures 6A and 6B illustrate several possible exemplary embodiments of the connecting side of the data acquisition means according to the invention, that enables the use of said means with different commercially available insulin pens;
- Figure 7 illustrates the pressure distribution by color coding within the material of the data acquisition means in use;
- Figure 8 is a semi-exploded perspective view of the data acquisition means according to the invention with the electronic subassembly;
- Figure 9 illustrates the electronic subassembly of the data acquisition means according to the invention with the push-button having an actuating function;
- Figure 10 is a block diagram of the electronic subassembly of the data acquisition means with power management function; and
- Figure 11 is a block diagram illustrating the operation of the data acquisition means.

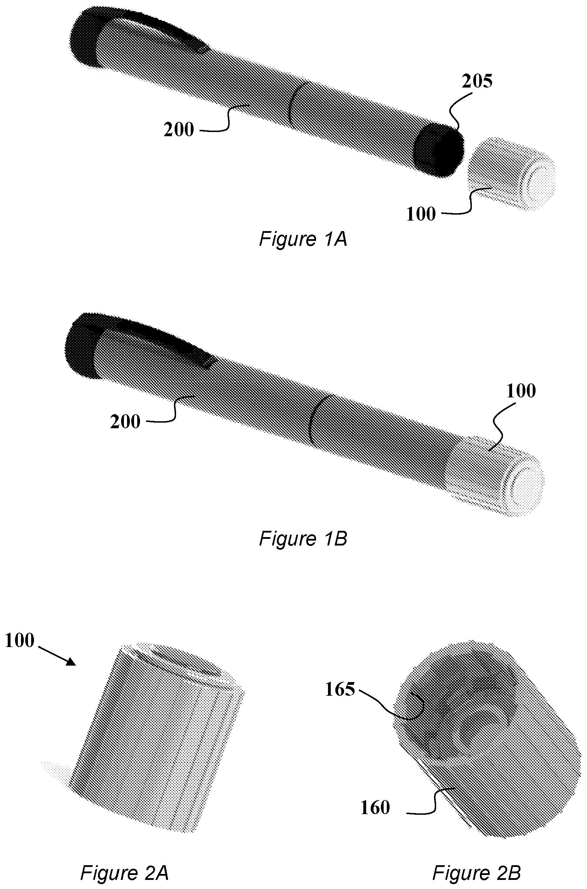

Figures 1A and 1B illustrate an exemplary insulin pen 200 and a respective data acquisition means 100 according to the invention, respectively, before assembly and after assembly. The data acquisition means 100 is fixed on the actuator member 205 of the pen 200, but can be removed from it by applying a force exceeding a certain threshold without damaging the insulin pen 200, the actuator member 205 or the data acquisition means 100. Construction and operation of said insulin pen 200 is known to a person skilled in the art, therefore is not discussed herein in more detail. Administration of insulin by the insulin pen 200 is performed through the end of the insulin pen 200 opposite to the one on which the actuator member 205 is arranged generally by actuating the actuator member 205, or here the data acquisition means 100. A preferred exemplary embodiment of the data acquisition means according to the invention is illustrated in Figures 2A and 2B in exploded view from the outside and from below, respectively. It can be seen in Figure 2B that inner surface 165 of mounting ring 160 assuring the fixed connection between the insulin pen 200 and the data acquisition means 100 is constructed as a structured surface that is complementary to the outer surface of the actuator member 205. Thus, when the data acquisition means 100 is mounted onto the insulin pen 200, a frictional and/or a form-fitting connection is established between the mounting ring 160 and the actuator member 205 which keeps the data acquisition means 100 continuously on the actuator member 205.

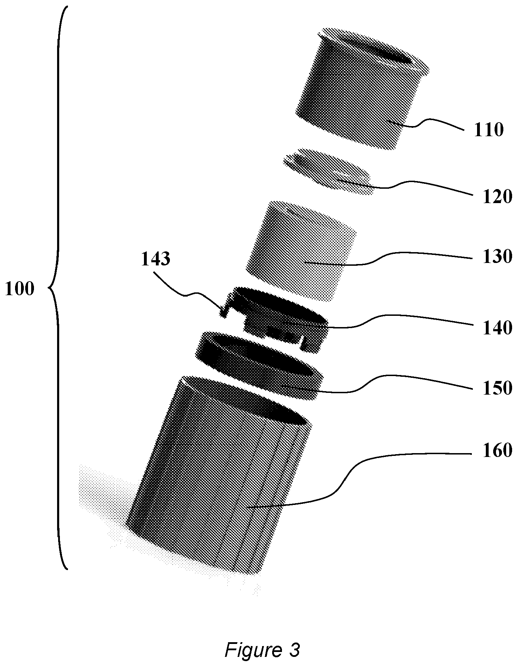

Figure 3 is an exploded view of the modular data acquisition means 100. Here, various parts of said means can be seen, such as the mounting ring 160 that forms a housing, and a button body 110, a push-button 120 being transparent to light on at least a portion thereof, an electronic subassembly 130, a button base 140 and a cover ring 150, all disposed within the housing. The button body 110, the push-button 120, the electronic subassembly 130 and the button base 140 form together a detector unit. Said parts, assembled into said detector unit, can be fitted in the mounting ring 160 that can be mounted onto a desired type of insulin pen. The detector unit is fitted in and secured to the mounting ring 160 by a snap-fit connection, which forms between legs 143 on the bottom side of the button base 140 and a cylindrical shoulder 163 on the inside surface of the mounting ring 160. Pressing the push-button 120 of the detector unit causes the push-button 120 to abut the sensor element 132, thereby generating a piece of information related to the actuation of the actuator member 205; said piece of information is transmitted then to the electronic subassembly 130, wherein it gets stored. At the same time, pushing the push-button 120 causes the insulin pen 200 to perform an insulin dispensing action due to mechanical transmission of the detector unit.

Figure 4 is a sectional view of an assembled data acquisition means 100, which shows that the cover ring 150 actually fills the space created between the mounting ring 160 and the button body 110 by joining together said unit and the mounting ring and thus it improves mechanical durability of the data acquisition means. After mounting the said means 100 onto the actuator member 205, the function of said actuator member (in particular, the push-button) of the insulin pen is taken over by the electronic subassembly 130 including electronics, while the mounting ring 160 and the bottom surface 145 of the button base 140 establish mechanical connection with the actuator member 205 of the insulin pen. Furthermore, the electronic subassembly 130 is received within the mounting ring 160.

The primary design requirement of the electronic subassembly 130 is the smallest possible size, since the size of the data acquisition means 100 have to be as small as possible, which is affected primarily by the outer dimensions of the electronic subassembly 130. The electronic subassembly 130 is disposed in a separate storage case, which is the same for every pen, independent of the type of the insulin pen. Said case of the electronic subassembly 130 can be fitted to the mounting ring 160. When the data acquisition means 100 is assembled, the electronic subassembly 130 is disposed in a cavity defined by the button body 110 and the button base 140.

The function of the mounting ring 160 is to establish connection between the insulin pen and the storage case of the electronic subassembly 130. The outer dimensions of the mounting rings 160 are the same, independent of the type of the pen. This requirement is necessary for the device to fit into a communication storage case, as in this way a single kind of storage case can be used, which reduces costs and makes the product and its use simpler. The mounting rings 160 have the same outer design independent of the type of the pen, thus the users have to get accustomed to/to learn practically the usage of one single product independent of the type of the insulin dispenser they use.

Figures 6A and 6B illustrate several exemplary embodiments of the inner (connecting) surface that attributes the data acquisition means according to the invention and the mounting ring 160 with the capability of being used in combination with different types of commercially available insulin pens (see Figure 5). The figures show the connection surfaces of the mounting rings 160 articulated by ribs and recesses separating said ribs; a complementary surface for forming connection with the desired type of insulin pen is obtained in this manner. As can also be seen in Figures 6A and 6B, the outer dimensions of the preferably cylindrical mounting rings 160 are the same, both in diameter and height, independent of the type of the insulin pen to be used, there are differences in shape only over the inner (connection) surface.

Said mounting rings 160, similarly to other plastic parts of the data acquisition means 100, are manufactured by polymer injection molding. A great deal of attention has to be paid to the design of internal and external surfaces. Undercuts are to be avoided when making the molds to keep costs low, because undercuts would anyway greatly increase the costs of the molds: the costs of a mold with undercut can be twice of the costs of a mold with no undercut. As a result, the inner geometry of the mounting rings 160 is somewhat more complicated, but can be manufactured by simpler and thus cheaper molds. Main considerations for the design of ribs on the outer cylindrical surface of the mounting rings 160 are ergonomic shape, easy handling and creation of an injection moldable structure.

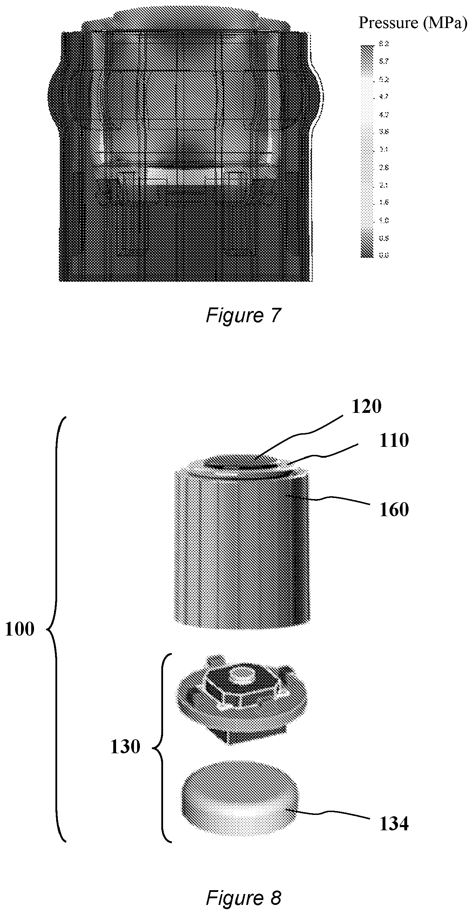

Figures 8 to 10 show the structural and block diagram of the electronic subassembly 130 of the data acquisition means according to the invention. The electronic subassembly 130 stores the instant and the duration of the administration, which is suitable for determining the amount of administered insulin, and transmits the stored data to the communication unit of the storage case through a wireless communication channel. The electronic subassembly 130 comprises LEDs 131, sensor element 132, particularly a pressure sensor, a quartz or other resonator 133 for providing clock signal, a power source 134 (preferably a battery, in particular, a button cell), a processor 135 and a wireless (preferably infrared) communication unit 136. The power source 134 is preferably fixedly built into the electronic subassembly 130, and therefore cannot be replaced. The LEDs 131 comprise LEDs 131A emitting preferably red light and LEDs 131B emitting preferably green light. The electronic subassembly 130 also comprises an integrated circuit board 137 that also serves as a base and supports the aforementioned electronic parts, as well as provides the electrical connection between said parts.

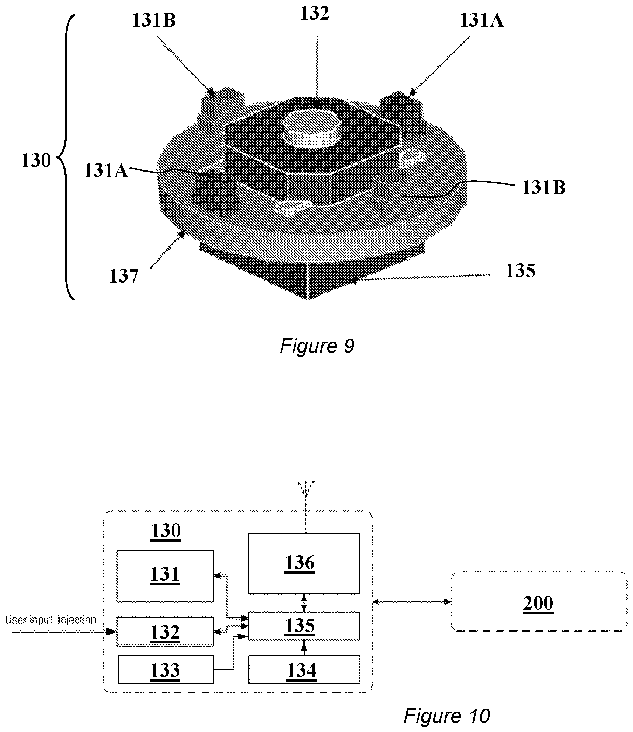

When technical design of the data acquisition means was under progress, the most important issue was to achieve a construction with the smallest possible size along with reliable load-resistance. The insulin dispensing pen and the data acquisition means are used one to four times a day, i.e. about 1000 times a year on average. Therefore, in a preferred exemplary embodiment, the data acquisition means also has a memory unit (not shown in Figure 9) - with a capacity of preferably 32 kB - that is capable of receiving and storing/preserving data related to each insulin administration (about 1000) performed during the lifetime of the insulin pen (i.e. about one year). The memory unit preferably preserves the stored information even in case of total power cut, it is provided e.g. by a 'non-volatile' memory, such as a flash memory. The processor 135 is preferably a 16-bit processor.

Technical design of the two major parts of the data acquisition means (the mounting ring 160 and the electronic subassembly 130) sets substantially different criteria. The electronic subassembly includes the printed circuit board 137 with a few small-sized parts arranged thereon and the small-sized power source 134. Besides small size, easy manufacturability has also been taken into account in the technical design process. The electronic subassembly 130 withstands to significant mechanical loads on the long term and operates in a stable manner. The part of the data acquisition means matching the insulin pen has got only mechanical functions and is subject merely to mechanical load. In the technical design process, the two most important considerations are the material selection and to achieve accurate matching. The applied materials are able to bear high physical load with no permanent deformation and weakening. Accurate matching is a fundamental requirement for usability and reliability of the data acquisition means. The data acquisition means according to the invention fits stably onto the pen, it does not fall from it, and thus the patient cannot lose it in everyday use.

The data acquisition means is designed by a 3D computer modeling program, which allows finite element simulations to be carried out for the model. This is suitable to predict the behavior of the data acquisition means in everyday use, thus any accidental errors can be still corrected before manufacturing. Results of the simulation show that a load of about 100 N (which is about the force that an adult exerts when pressing the push-button 120 of the data acquisition means to the sensor element 132, in particular, the pressure sensor) causes a deformation of about 0.02 mm in the material, which is negligible. The same force causes a tension of 6.2 MPa in the material (see Figure 7), which is also negligible compared to the 15 MPa yield point of the polymer material of the button base 140.

Accordingly, the data acquisition means according to the invention has the ability to entirely transfer the force exerted on the push-button 120 by the user to the actuator member 205 of the insulin pen via the button base 140. This is important and preferred, as in this way the data acquisition means 100 does not inhibit insulin administration, and after mounting the data acquisition means 100 onto the insulin pen, the proper operation of said insulin pen does not require significantly larger force. This feature would be adversely affected by an undesired deformation arising (either at each press of the button and/or on the long term) in use. The materials of the parts of the data acquisition means required for a flawless operation are preferably selected from polymers by taking the aforementioned considerations into account.

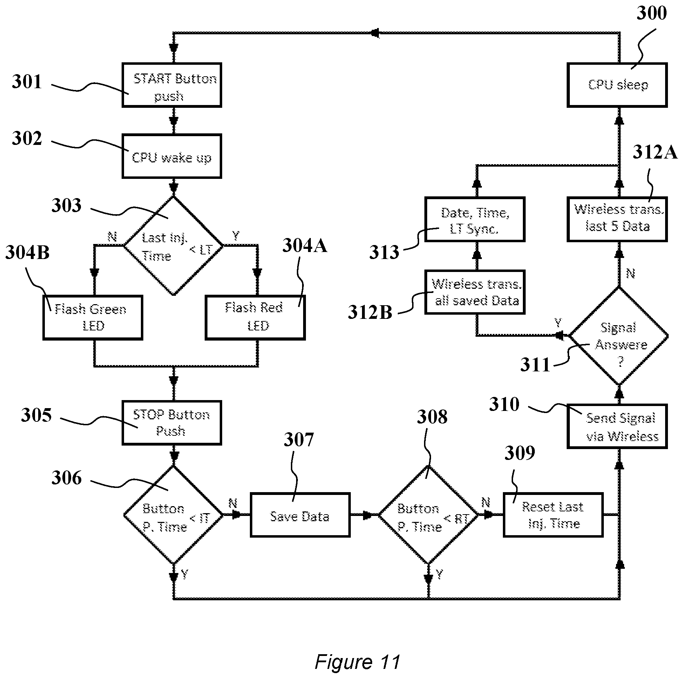

In what follows, operation of an exemplary embodiment of the data acquisition means according to the invention is described briefly with reference to the block diagram shown in Figure 11.

To ensure low power consumption, the electronic subassembly 130 of the data acquisition means 100 according to the invention contains the most modern surface mounted device (SMD) components. Moreover, to increase the lifetime of the button cell battery, the consumption is optimized by software. As a result, the data acquisition means 100 becomes suitable for serving the insulin pen used in combination therewith during its whole lifetime.

In its default state, at least a part of the data acquisition means, i.e. its processor (CPU) is in sleeping mode (300). The processor is activated (301) when the push-button of said means is pressed. If this takes place, the data acquisition means tests (303) whether the time passed since the last insulin administration is longer or shorter than a predetermined first period of time LT - which is preferably 3 hours. If it is shorter, the data acquisition means provides a first response (304A) to the user in the form of a predetermined number of flashes of a preferably red LED, which indicates that insulin administration is not recommended yet. If more time passed than said predetermined first time period LT, the data acquisition means provides the user with a second response (304B) in the form of a predetermined number of flashes of a preferably green LED, which indicates that sufficient amount of time has already passed since the last insulin administration to effect the next insulin administration. The predetermined number of flashes of red/green LED(s) are adjusted in compliance with the charge level of the internal power source of the data acquisition means; at full charge level of the battery, the number of flashes will be a larger number, e.g. at least five flashes are performed, while if the charge level of the power source falls under a first threshold of charge level - e.g. 30% of the nominal full charge - said number will be a smaller number, e.g. three. If charge level of the battery is reduced below a second threshold of charge level - e.g. 10% of the nominal full charge - the number of flashes by the LED(s) will be an even smaller number, e.g. 0, that is, the LED(s) will no longer flash. This also indicates to the user that the power source is expected to run down. Upon this indication, the user can take care of saving the data stored in the data acquisition means and obtaining a new data acquisition means, if necessary.

The number of flashes performed by the red and the green LEDs can be equal or different. The electronic subassembly 130 comprises one or more, preferably two pieces of red/green LEDs. If the data acquisition means comprises more than one LEDs of the same color, the LEDs of same color can be flashed simultaneously or alternately. In order to indicate the fact as harshly as possible that insulin administration is not recommended when the LED emits in red, alternate flashing of the LEDs is highly preferred.

When the push-button is released (305), the device tests (306) if the duration of holding down the button was longer or shorter than a second predetermined period IT (which is preferably e.g. 0.3 seconds). If it is longer, the data acquisition means maps the pressing of the button with an insulin administration event, and stores (307) the duration and the instant of pressing the button in the memory unit. If it is shorter, said means continues its operation by skipping at least one step and at most as many steps as still remain until returning to the sleeping mode, preferably by skipping more than one step, and most preferably by skipping the three steps (steps 307 to 309) following the test.

Optionally, the data acquisition means is waiting for several (e.g. 15) seconds after releasing the push-button - before or after data storing (307) - and if the button is pressed again, and the duration of this pressing down is longer than the second predetermined period, then (if the waiting period comes before data storing (307)) it adds its duration to the previous duration of button pressing down and stores the sum of the two durations, or (if the waiting period comes after data storing (307)) it stores said duration as a new administration duration.

After storing (307) the duration of pressing down the button which is deemed to correspond to an insulin administration, the data acquisition means tests (308) if the duration of holding down the button is longer or shorter than a third period RT. If it is longer, the said means adjusts (309) the instant of last insulin administration to the instant of the actual button press. If it is shorter, the said means continues to operate by skipping at least one and at most as many steps as remain until returning to the sleeping mode, preferably by skipping one step (i.e. adjusting (309) of the instant of last insulin administration).

After this, the data acquisition means transmits (310) a wireless communication signal and tests for a given period of time if a response signal is received, i.e. it is waiting (311) for a response signal. If no response signal is received within the given period of time, it transmits (312A) data related to the at least one, preferably five latest insulin administrations. The data acquisition means then repeats the signal transmission (310), the data transmission (312A) at least once, preferably e.g. 30 times, and/or performs further steps after further delay of preferably one minute. If a response signal is received, the data acquisition means transmits (312B) all the stored data and, optionally, based on the response signal received it adjusts (313) the date, the instant and/or one or more of the predetermined periods, preferably the first predetermined period.

After this, the data acquisition means returns to sleeping mode (300) in order to spare with the power source.

The processor 135 is in sleeping mode most of the time, therefore its consumption is low. The activated LEDs' power consumption is the highest, ∼5 mA. The program code requires a relatively short time for its execution. By keeping the number of executions low, the long term consumption also stays low. The lowest possible consumption is achieved in the sleeping mode, wherein it is 0.4 mA. Consequently, the data acquisition means according to the invention can operate with a power source provided by a button cell for about 1.5 years, which is more than the lifetime of most insulin pens (with replaceable cartridges), which is about 1 year. Consequently, due to the energy management scheme described hereinbefore, the data acquisition means according to the invention can reliably operate through its whole lifetime with the battery inserted into it when it has been manufactured. Furthermore, when the insulin pen is replaced (either because the physician prescribes a new one or due to inevitable wearing), the patient preferably receives a new data acquisition means. Hence, there is no need to replace the power source of the electronic subassembly 130 during the lifetime of the insulin pen, and thus while maintaining reliable operation, manufacturing costs of the data acquisition means according to the invention can be further reduced by making use of a built-in power source.

When the power source is low on charge, preferably at about 30% of its nominal charge, to save power the data acquisition means switches to a power saving mode upon instructed to do so by the processor. Power saving operation includes - besides maintaining other functions of the device unchanged - that the LEDs will flash fewer times to inform the user. Furthermore, when reaching an even lower charge level of the power source, e.g. about 10% of the nominal charge, upon instructed by the processor, the data acquisition means switches to an operational state in which even more power can be saved. In this state the LEDs will no longer flash when the insulin pen is used, but at least one of its other functions, preferably all of its other functions remain constantly available. Now, the lack of flashing of the LEDs also indicates to the user that the power source is about to run down.

In a preferred embodiment, the data acquisition means is used in combination with an additional communication device, e.g. a storage case. In such a case the repeated and delayed signal transmission ensures that signal transmission also takes place at that instant when said data acquisition means and the insulin pen have already been put back into the case - this usually takes place in less than 30 minutes. The storage and transmission of the pieces of information regarding more than one insulin administration by the data acquisition means practically ensures that said means transmits every relevant piece of information to the communication storage case when it is put back into the case, even though it was removed from the case for a longer period of time (e.g. 1 day). The data acquisition means is configured to allow re-set of the date/time and optionally some additional operation parameters, e.g. the first predetermined period (which is the threshold period belonging to the signal that warns the user if a repeated insulin administration is intended to be performed too early) upon detecting incoming communication. This allows the use of data acquisition means with the same factory settings to be used in combination with insulin pens of different kind - i.e. containing long-acting base insulin or short-acting insulin - and in such cases it also allows to re-set the period of warning, if necessary.

In a preferred embodiment, the data acquisition means comprises an electronic oscillator (e.g. an RC circuit), preferably with an oscillator having a quartz crystal, thus the error of timekeeping remains low from the startup of said means to the end of its lifetime.

## Claims

1. A data acquisition means (100) to be used with a drug delivery device (200) for continuously acquiring information about operation of the device (200) during the lifetime of the device (200) and for indicating the information about the operation of the device (200) to a user, comprising
- a detector unit having a sensor element (132), said sensor element (132) is configured to detect an actuation action to be performed on an actuator member (205) of the drug delivery device (200) in order to dispense at least a portion of a drug contained within the drug delivery device (200), and a push-button (120) configured to exert said actuation action on said sensor element (132) by pressing said push-button (120), wherein said sensor element (132) is configured to detect said actuation action as pressing the push-button (120) as part of said actuation action; and
- an electronic subassembly (130) having a power source (134), said electronic subassembly (130) being connected to the sensor element (132) and to the power source (134), wherein the electronic subassembly (130) is configured to store at least a piece of information related to the detected actuation action,
**characterized in that**
the electronic subassembly (130) is further configured to transmit signal(s) related to said piece(s) of information to the user, the at least one signal being in compliance with an actual charge level of the power source (134) and changing as the charge level of said power source (134) changes.

2. The data acquisition means (100) according to claim 1, further comprising a mounting ring (160), configured to provide a detachable connection of the data acquisition means (100) with the actuator member (205) of the drug delivery device (200).

3. The data acquisition means (100) according to claim 2, wherein the detector unit, the electronic subassembly (130) and the mounting ring (160) forms an integral unit, when assembled.

4. The data acquisition means (100) according to claim 2, wherein said connection of the data acquisition means (100) and the actuator member (205) is formed by a frictional and/or form-fitting connection between the actuator member (205) and the internal surface of the mounting ring (160).

5. The data acquisition means (100) according to any one of claims 1 to 4, wherein the electronic subunit has at least one optical element adapted to emit light, and wherein the signal related to the information related to the detected actuation action is transmitted by said optical element.

6. The data acquisition means (100) according to claim 5, wherein the at least one optical element adapted to emit light is provided by a combination of at least one light emitting diode adapted to emit red light and at least one light emitting diode adapted to emit green light.

7. The data acquisition means (100) according to claim 5 or 6, wherein transmitting signal(s) related to the piece(s) of information related to the detected actuation action in compliance with the actual charge level of the power source (134) takes place by adjusting the number of optical emissions by said at least one optical element in compliance with the charge level of the power source (134).

8. The data acquisition means (100) according to claim 7, wherein the number of optical emissions by said at least one optical element is a first number if the actual charge level of said power source (134) is above a first threshold, and wherein the number of optical emissions is a second number if the actual charge level of said power source (134) is between the first threshold and a second threshold, said second threshold being smaller than said first threshold, and wherein the number of optical emissions is a third number if the actual charge level of said power source (134) is below the second threshold, wherein said first number is larger than the second number, and said second number is larger than the third number.

9. The data acquisition means (100) according to claim 8, wherein the third number is zero providing indication of an expected running down of the power source (134).

10. The data acquisition means (100) according to any of the preceding claims, wherein the drug delivery device (200) is a pen-type insulin dispensing device.

## Patentansprüche

1. Datenerfassungsvorrichtung (100), die mit einer Medikamentenabgabevorrichtung (200) verwendet wird, um kontinuierlich Informationen über den Betrieb der Vorrichtung (200) während der Lebensdauer der Vorrichtung (200) zu erfassen und einem Benutzer die Informationen über den Betrieb der Vorrichtung (200) anzuzeigen, umfassend
- eine Detektoreinheit mit einem Sensorelement (132), wobei das Sensorelement (132) konfiguriert ist, um eine Betätigungsaktion zu erfassen, die an einem Betätigungselement (205) der Medikamentenabgabevorrichtung (200) durchzuführen ist, um mindestens einen Teil eines in der Medikamentenabgabevorrichtung (200) enthaltenen Medikaments abzugeben, und eine Drucktaste (120), die konfiguriert ist, um die Betätigungsaktion auf das Sensorelement (132) durch Drücken der Drucktaste (120) auszuüben, wobei das Sensorelement (132) konfiguriert ist, um die Betätigungsaktion als Drücken der Drucktaste (120) als Teil der Betätigungsaktion zu erfassen; und
- eine elektronische Unterbaugruppe (130) mit einer Stromquelle (134), wobei die elektronische Unterbaugruppe (130) mit dem Sensorelement (132) und der Stromquelle (134) verbunden ist, wobei die elektronische Unterbaugruppe (130) konfiguriert ist, um mindestens eine Information bezüglich der erfassten Betätigungsaktion zu speichern,
**dadurch gekennzeichnet, dass**
die elektronische Unterbaugruppe (130) weiter konfiguriert ist, um Signal(e), die sich auf die Informationen beziehen, auf den Benutzer zu übertragen, wobei das mindestens eine Signal mit einem tatsächlichen Ladezustand der Stromquelle (134) übereinstimmt und sich mit der Änderung des Ladezustands der Stromquelle (134) ändert.

2. Datenerfassungsvorrichtung (100) nach Anspruch 1, weiter umfassend einen Montagering (160), der konfiguriert ist, um eine lösbare Verbindung der Datenerfassungsvorrichtung (100) mit dem Betätigungselement (205) der Medikamentenabgabevorrichtung (200) bereitzustellen.

3. Datenerfassungsvorrichtung (100) nach Anspruch 2, wobei die Detektoreinheit, die elektronische Unterbaugruppe (130) und der Montagering (160) im montierten Zustand eine integrierte Einheit bilden.

4. Datenerfassungsvorrichtung (100) nach Anspruch 2, wobei die Verbindung der Datenerfassungsvorrichtung (100) und des Betätigungselements (205) durch eine reibungs- und/oder formschlüssige Verbindung zwischen dem Betätigungselement (205) und der Innenfläche des Montagerings (160) gebildet ist.

5. Datenerfassungsvorrichtung (100) nach einem der Ansprüche 1 bis 4, wobei die elektronische Untereinheit mindestens ein optisches Element aufweist, das geeignet ist, Licht zu emittieren, und wobei das Signal, das sich auf die Informationen bezieht, die sich auf die erfasste Betätigungsaktion beziehen, von dem optischen Element übertragen wird.

6. Datenerfassungsvorrichtung (100) nach Anspruch 5, wobei das mindestens eine optische Element, das zum Aussenden von Licht geeignet ist, durch eine Kombination aus mindestens einer lichtemittierenden Diode, die zum Aussenden von rotem Licht geeignet ist, und mindestens einer lichtemittierenden Diode, die zum Aussenden von grünem Licht geeignet ist, bereitgestellt wird.

7. Datenerfassungsvorrichtung (100) nach Anspruch 5 oder 6, wobei das Übertragen von Signal(en), die sich auf Informationen beziehen, die sich auf die erfasste Betätigungsaktion in Übereinstimmung mit dem tatsächlichen Ladezustand der Stromquelle (134) beziehen, durch Einstellen der Anzahl der optischen Emissionen durch das mindestens eine optische Element in Übereinstimmung mit dem Ladezustand der Stromquelle (134) erfolgt.

8. Datenerfassungsvorrichtung (100) nach Anspruch 7, wobei die Anzahl der optischen Emissionen durch das mindestens eine optische Element eine erste Zahl ist, wenn der tatsächliche Ladezustand der Stromquelle (134) über einem ersten Schwellenwert liegt, und wobei die Anzahl der optischen Emissionen eine zweite Zahl ist, wenn der tatsächliche Ladezustand der Stromquelle (134) zwischen dem ersten Schwellenwert und einem zweiten Schwellenwert liegt, wobei der zweite Schwellenwert kleiner als der erste Schwellenwert ist, und wobei die Anzahl der optischen Emissionen eine dritte Zahl ist, wenn der tatsächliche Ladezustand der Stromquelle (134) unter dem zweiten Schwellenwert liegt, wobei die erste Zahl größer als die zweite Zahl ist und die zweite Zahl größer als die dritte Zahl ist.

9. Datenerfassungsvorrichtung (100) nach Anspruch 8, wobei die dritte Zahl Null ist, die einen Hinweis auf ein erwartetes Herunterfahren der Stromquelle (134) liefert.

10. Datenerfassungsvorrichtung (100) nach einem der vorstehenden Ansprüche, wobei die Medikamentenabgabevorrichtung (200) eine Insulindosiervorrichtung vom Stifttyp ist.

## Revendications

1. Moyen d'acquisition de données (100) à utiliser avec un dispositif d'administration de médicament (200) pour acquérir en continu des informations sur le fonctionnement du dispositif (200) pendant la durée de vie du dispositif (200) et pour indiquer les informations relatives au fonctionnement du dispositif (200) à un utilisateur, comprenant
- une unité de détection présentant un élément de capteur (132), ledit élément de capteur (132) est configuré pour détecter une action d'actionnement devant être effectuée sur un élément d'actionneur (205) du dispositif d'administration de médicament (200) afin de distribuer au moins une partie d'un médicament contenu dans le dispositif d'administration de médicament (200), et un bouton-poussoir (120) configuré pour exercer ladite action d'actionnement sur ledit élément de capteur (132) en appuyant sur ledit bouton-poussoir (120), dans lequel ledit élément de capteur (132) est configuré pour détecter ladite action d'actionnement en appuyant sur le bouton-poussoir (120) dans le cadre de ladite action d'actionnement ; et
- un sous-ensemble électronique (130) présentant une source d'alimentation (134), ledit sous-ensemble électronique (130) étant connecté à l'élément de capteur (132) et à la source d'alimentation (134), dans lequel le sous-ensemble électronique (130) est configuré pour stocker au moins une pièce d'information liée à l'action d'actionnement détectée,
**caractérisé en ce que**
le sous-ensemble électronique (130) est en outre configuré pour transmettre à l'utilisateur un ou des signaux associés à ladite ou auxdites pièces d'information, le au moins un signal étant conforme à un niveau de charge actuel de la source d'alimentation (134) et changeant lorsque le niveau de charge de ladite source d'alimentation (134) change.

2. Moyen d'acquisition de données (100) selon la revendication 1, comprenant en outre une bague de montage (160) configurée pour fournir une connexion détachable du moyen d'acquisition de données (100) avec l'élément d'actionneur (205) du dispositif d'administration de médicament (200).

3. Moyen d'acquisition de données (100) selon la revendication 2, dans lequel l'unité de détecteur, le sous-ensemble électronique (130) et la bague de montage (160) forme une unité d'un seul tenant, une fois assemblés.

4. Moyen d'acquisition de données (100) selon la revendication 2, dans lequel ladite connexion du moyen d'acquisition de données (100) et de l'élément d'actionneur (205) est formée par une connexion par friction et/ou par ajustement de forme entre l'élément d'actionneur (205) et la surface interne de la bague de montage (160).

5. Moyen d'acquisition de données (100) selon l'une quelconque des revendications 1 à 4, dans lequel la sous-unité électronique présente au moins un élément optique adapté pour émettre de la lumière, et dans lequel le signal lié à l'information relative à l'action d'actionnement détectée est transmis par ledit élément optique.

6. Moyen d'acquisition de données (100) selon la revendication 5, dans lequel le au moins un élément optique adapté pour émettre de la lumière est fourni par une combinaison d'au moins une diode électroluminescente adaptée pour émettre de la lumière rouge et d'au moins une diode électroluminescente adaptée pour émettre de la lumière verte.

7. Moyen d'acquisition de données (100) selon la revendication 5 ou 6, dans lequel la transmission du ou des signaux lié(s) au(x) pièce(s) d'information relatives à l'action d'actionnement détectée en conformité avec le niveau de charge actuelle de la source d'alimentation (134) intervient en ajustant le nombre d'émissions optiques par ledit au moins un élément optique en conformité avec le niveau de charge de la source d'alimentation (134).

8. Moyen d'acquisition de données (100) selon la revendication 7, dans lequel le nombre d'émissions optiques par ledit au moins un élément optique est un premier nombre si le niveau de charge actuel de ladite source d'alimentation (134) est supérieur à un premier seuil, et dans lequel le nombre d'émissions optiques est un deuxième nombre si le niveau de charge actuel de ladite source d'alimentation (134) est compris entre le premier seuil et un second seuil, ledit second seuil étant inférieur audit premier seuil, et dans lequel le nombre d'émissions optiques est un troisième nombre si le niveau de charge actuel de ladite source d'alimentation (134) est inférieur au second seuil, dans lequel ledit premier nombre est supérieur au deuxième nombre, et ledit deuxième nombre est supérieur au troisième nombre.

9. Moyen d'acquisition de données (100) selon la revendication 8, dans lequel le troisième nombre est égal à zéro et fournit une indication d'un arrêt attendu de la source d'alimentation (134).

10. Moyen d'acquisition de données (100) selon l'une quelconque des revendications précédentes, dans lequel le dispositif d'administration de médicament (200) est un dispositif de distribution d'insuline du type stylet.
